# EUROPÄISCHE PATENTSCHRIFT

(11) **EP 3 138 730 B1**
(45) Veröffentlichungstag und Bekanntmachung des Hinweises auf die Patenterteilung: **07.08.2019**
(21) Anmeldenummer: 16173645.9
(22) Anmeldetag: 09.06.2016
(51) Int. Cl.: B60N 2/56, B60N 2/58, A47G 9/02, A61F 7/00

(54) **TEMPERIERBARE SOFT-TOUCH-POLSTERSTRUKTUR**
MULTILAYER SOFT TOUCH CUSHION STRUCTURE WITH A CONTROL OF THE TEMPERATURE
STRUCTURE DE REMBOURRAGE MULTICOUCHE A TOUCHER DOUX ET THERMOREGULEE

(30) Priorität: 03.09.2015 DE 102015216827
(43) Veröffentlichungstag der Anmeldung: 08.03.2017
(73) Patentinhaber: Benecke-Kaliko AG, 30419 Hannover (DE)
(72) Erfinder: Kaschub, Dirk, 31275 Lehrte (DE); Gerken, Andreas, 30161 Hannover (DE)
(74) Vertreter: Kilsch, Armin Ralph

(56) Entgegenhaltungen:
- WO-A1-98/55340
- DE-T2- 69 705 910
- JP-A- 2014 159 264

## Beschreibung

Die Erfindung betrifft eine temperierbare mehrschichtige Soft-Touch-Polsterstruktur oder dergleichen, umfassend eine im Wesentlichen gasundurchlässige Obermaterialschicht und zumindest eine unter dieser angeordnete voluminöse, druckelastische Untermaterialschicht zum Durchführen eines Temperierens.

Der Begriff "Soft-Touch-Polsterstruktur" oder "Polsterstruktur mit weicher Oberfläche" bezeichnet insbesondere im Automobilbau Polsterstrukturen und Formteilverkleidungen mit einem mehrschichtigen, druckelastischen Aufbau sowie mit zumindest mit einer üblicherweise gasundurchlässigen Obermaterialschicht. Diese Polsterstrukturen und Formteilverkleidungen weisen neben einer guten Verschleißfestigkeit auch dekorative Elemente auf. Sie sollen angenehme Berührungseigenschaften haben sowie unter der Obermaterialschicht angeordnet eine voluminöse, druckelastische Untermaterialschicht aufweisen, welche der gesamten Struktur die erwünschte Soft-Touch-Haptik verleiht. Derartige Polsterstrukturen und Formteilverkleidungen werden beispielsweise für Säulen- und Türverkleidungen, als Dachhimmel oder auch für Sitzpolsterungen im Fahrzeugbau eingesetzt.

Ein Nachteil bekannter Strukturen der beschriebenen Art wird darin gesehen, dass die üblicherweise aus Vliesen oder Schäumen bestehende Untermaterialschicht zumindest weitgehend luftundurchlässig ist oder nur einen unkontrollierten und deshalb nicht nutzbaren Luftdurchsatz erlaubt. Es werden demnach lediglich die mechanischen Eigenschaften der Untermaterialschicht genutzt.

Aus der DE 10 2008 019 045 A1 ist zwar bereits eine Polsterstruktur für einen Autositz bekannt, bei welchem ein die Sitzfläche oder Lehnenfläche bildender voluminöser Schaumstoff mit in dessen Oberfläche ausgebildeten Kanälen ausgestattet ist, die einerseits Auflagebereiche mit unterschiedlicher Härte herstellen und andererseits ein Schlauchsystem aufnehmen sollen, das mit einem heiz- oder kühlbaren Medium füllbar ist. Dieses System ist jedoch wegen der Bearbeitung der Schaumstoffoberflächen und wegen der zusätzlichen Installation des Schlauchsystems sowie der diese mit dem Medium versorgenden Befüllungseinrichtung sehr aufwendig und teuer in der Herstellung.

Aus der DE 100 24 879 C1 ist ein aktiv belüftetes Sitzmodul für einen Fahrzeugsitz bekannt, bei welchem die der oben beschriebenen Untermaterialschicht entsprechende untere Polsterschicht luftundurchlässig ist, jedoch von mit Miniaturlüftern ausgestatteten Kanälen durchsetzt und mit einer luftdurchlässigen mittleren Luftführungsschicht sowie einer luftdurchlässigen oberen Polsterschicht überdeckt ist, welche die von den Miniaturlüftern punktuell erzeugte Luftströmung verteilen und einem weiteren, darüber angeordneten Polsterbezug zum Zwecke einer Sitzbelüftung zuführen. Zusätzlich kann unterhalb des Polsterbezuges eine Sitzheizung vorgesehen sein. Trotz des äußerst aufwendigen und teuren Aufbaus dieser Anordnung kann diese allenfalls eine Belüftung und Heizung, jedoch keine Sitzkühlung gewährleisten.

Die DE 697 05 910 T2 offenbart eine Lüftungsanlage zur Verwendung mit einem Fahrzeugsitz, die ein Luftkanalelement aufweist, dessen Oberfläche im Wesentlichen luftundurchlässig ist, jedoch einzelne luftdurchlässige Bereiche aufweist, soweit die Sitzfläche betroffen ist. Eine Unterkonstruktion bzw. Luftlage oder Kissenelement ist mit Luft durchströmbar, wobei sich nur an den Vorderflächen der Unterkonstruktion Öffnungen zum Lufteintritt und -austritt befinden, die an ein Luftkanalelement angeschlossen werden können. Nachteilig an dieser Konstruktion ist es, dass die luftdurchlässige Fläche rund um den durchströmbaren Bereich bis auf die Ein- und Austrittsöffnungen luftdicht abgeschlossen werden muss.

Die WO 98/55340 A1 offenbart eine Sitzklimatisierungsvorrichtung, die aus einem mattenartig ausgebildeten, luftdurchlässigen Grundkörper besteht, etwa aus einem Abstandsgewirke oder aus einem Monofill-Gewirr, der von einem ebenfalls mattenartig ausgebildeten Heizelement durchzogen ist. Der Grundkörper kann dabei mit einer wärmereflektierenden Schicht bezogen sein. Dadurch ist zwar eine schnelle Beheizung eine Sitzfläche möglich, aber nachteiliger Weise keine Kühlung gewährleistet.

Vor diesem Hintergrund liegt der Erfindung die Aufgabe zugrunde, eine Polsterstruktur zu schaffen, welche mit einfachen konstruktiven Mitteln ein Heizen und Kühlen sowie gemäß einer weiteren Ausgestaltung ein Belüften dieser Polsterstruktur und damit letztlich auch deren Umgebung erlauben.

Die Lösung dieser Aufgabe ergibt sich aus den Merkmalen des unabhängigen Patentanspruchs, während vorteilhafte Ausgestaltungen und Weiterbildungen der Erfindung den jeweils nachgeordneten Unteransprüchen entnehmbar sind.

Der Erfindung liegt die Erkenntnis zugrunde, dass eine Polsterstruktur der genannten Art bei einer entsprechenden Ausgestaltung mittels eines direkt in die Polsterstruktur einleitbaren Heizmediums oder Kühlmediums temperierbar sein sollte.

Demnach geht die Erfindung zunächst aus von einer mehrschichtigen Soft-Touch-Polsterstruktur oder dergleichen, welche eine im Wesentlichen gasundurchlässige Obermaterialschicht und zumindest eine unter dieser angeordnete voluminöse, druckelastische Untermaterialschicht aufweist. Zur Lösung der gestellten Aufgabe ist dabei vorgesehen, dass durch die gasdurchlässig ausgebildete Untermaterialschicht ein in der Schichtebene der Untermaterialschicht strömfähiges gasförmiges Kühlmedium oder Heizmedium hindurch geleitet wird.

Das gasförmige Medium kann mittels herkömmlicher Klimatisierungseinrichtungen temperiert werden, beispielsweise mittels elektrischer Heiz- oder Pelletierelemente. Im Automobilbau stehen dafür die Innenraumheizung und die Klimaanlage zur Verfügung.

Zur Lösung der Aufgabe ist vorgesehen, dass die Untermaterialschicht aus einem gasdurchlässigen Material besteht, dass die Unterseite der Untermaterialschicht mit einer gasundurchlässige Wandfolie verschlossen ist, und dass in den einströmseitigen Stirnseitenbereich sowie in dem ausströmseitigen Stirnseitenbereich der Untermaterialschicht Zugangs- und Ausgangsöffnungen für ein durch die Untermaterialschicht hindurch zu leitendes gasförmiges Kühlmedium beziehungsweise Heizmedium vorgesehen sind.

Aus der DE 201 22 399 U1 ist zwar schon eine Soft-Touch-Polsterstruktur bekannt, bei der an der Unterseite der der vorliegenden Untermaterialschicht entsprechenden Mittelschicht eine Barrierefolie angeordnet ist. Diese dient jedoch ausschließlich dazu, zu verhindern, dass chemische Bestandteile beim Hinterspritzen mit Kunststoff oder Kleberbestandteile nicht in die Mittelschicht gelangen und deren mechanische Eigenschaften beeinflussen, während die Wandfolie gemäß der vorliegenden Erfindung die gasdurchlässige Struktur der Untermaterialschicht von innen nach außen abschließt.

Die Untermaterialschicht bildet auf diese Weise zwischen der im Wesentlichen gasundurchlässigen Obermaterialschicht und der gasundurchlässigen unteren Wandfolie einen in der Schichtebene der Untermaterialschicht verlaufenden Gaskanal, durch den das Kühlmedium oder Heizmedium, im allgemeinen Luft, geleitet werden kann. Mittels des auf die jeweils erwünschte Temperatur gekühlten oder geheizten gasförmigen Mediums kann die Obermaterialschicht unmittelbar in der gewünschten Weise temperiert werden. Auf diese Weise lassen sich beispielsweise mit der erfindungsgemäßen Polsterstruktur gepolsterte Sitze direkt beliebig temperieren oder ein mit dieser Polsterstruktur ausgekleideter Innenraum eines Fahrzeuges auf eine gewünschte Raumtemperatur bringen.

Andere Anwendungsbereiche der erfindungsgemäßen Polsterstruktur sind beispielsweise Ruheliegen, Krankenliegen oder Schutzkleidung für Arbeiten in extrem heißer oder kalter Arbeitsumgebung.

Erfindungsgemäß ist die Polsterstruktur schlauchförmig ausgebildet, so dass neben der Obermaterialschicht und der gasundurchlässigen Wandfolie keine gasundurchlässigen Seitenwände benötigt werden, um einen geschlossenen, in diesem Fall dann zylindrischen Strömungskanal für das durchzuleitende Kühlmedium oder Heizmedium auszubilden, der mit dem gasdurchlässigen Material der Untermaterialschicht gefüllt ist. Demnach ist erfindungsgemäß vorgesehen, dass die Polsterstruktur eine schlauchförmige Geometrie aufweist, wobei von radial außen nach radial innen gesehen zunächst die Obermaterialschicht, dann die Untermaterialschicht und schließlich die gasundurchlässige Wandfolie angeordnet ist.

Das gasförmige Medium wird jeweils über einen als Zugangsöffnung dienenden Stirnseitenbereich der Polsterstruktur in die Untermaterialschicht eingeleitet und verlässt diese über einen als Ausgangsöffnung dienenden Stirnseitenbereich der erfindungsgemäßen Polsterstruktur.

Gemäß einer weiteren Ausgestaltung der Erfindung ist die Polsterstruktur als Flächengebilde beziehungsweise bahnförmig ausgebildet, wobei die nicht als Zugangs- oder Ausgangsöffnungen vorgesehenen seitlichen Stirnseitenbereiche der Untermaterialschicht ebenfalls durch eine gasundurchlässige Wandfolie gasdicht verschlossen sind, um den Gaskanal auch seitlich zu begrenzen.

Die Obermaterialschicht der Polsterstruktur besteht beispielsweise aus Polyurethan (PUR), Polyvinylchlorid (PVC), Polyolefin (TPO), Leder oder Textilware. Es sind auch beliebige andere Materialien verwendbar, sofern diese die oben für die Obermaterialschicht genannten Eigenschaften aufweisen. Die Verbindung der Obermaterialschicht mit der Untermaterialschicht erfolgt beispielsweise durch Kaschieren oder Kleben.

Es sei hier erwähnt, dass die Obermaterialschicht auch gering gasdurchlässig sein kann, so dass ein Anteil des gasförmigen Mediums über die Obermaterialschicht austreten und damit einen Belüftungseffekt bewirken kann.

Die Untermaterialschicht besteht beispielsweise aus einem Abstandsgewirke, einem offenporigen Schaumstoff oder einer Vliesstruktur. Auch hierbei sind andere Materialien möglich, sofern sie die weiter oben für die Untermaterialschicht genannten Eigenschaften haben.

Gemäß einer weiteren Ausgestaltung der erfindungsgemäßen Polsterstruktur kann vorgesehen sein, dass zwischen der Obermaterialschicht und der Untermaterialschicht eine Textilträgerschicht, beispielsweise aus einem Baumwoll-Polyester-Material, einem Polyester-Material, einem Vlies oder einer Polyolefin-Folie (TPO-Folie) angeordnet ist. Die Anordnung einer solchen Textilträgerschicht kann sowohl bei der schlauchförmigen als auch bei der bahnförmigen Variante der Posterstruktur vorgesehen sein.

Eine weitere Ausgestaltung der Erfindung sieht vor, dass die an der Oberseite der Untermaterialschicht befindliche Schichtanordnung, also die Obermaterialschicht selbst oder eine aus einer Obermaterialschicht und einem Textilträger bestehende Struktur, perforiert ist, beispielsweise um eine diffuse Belüftung in einem Fahrzeuginnenraum zu erzielen, womit beispielsweise herkömmliche Luftdüsen in der Instrumententafel eines Fahrzeuges entbehrlich sind.

Die Erfindung wird nachfolgend anhand von Ausführungsbeispielen weiter erläutern. Dazu ist der Beschreibung eine Zeichnung beigefügt. In dieser zeigt
Fig. 1 in einer perspektivischen Darstellung eine schematische Draufsicht auf eine bahnförmige Soft-Touch-Polsterstruktur, die nicht Teil der vorliegenden Erfindung ist, ist, in einer Explosionsdarstellung,
Fig. 2 die Soft-Touch-Polsterstruktur gemäß der Fig. 1 in einer Seitenansicht,
Fig. 3 eine andere Ausgestaltung einer Soft-Touch-Polsterstruktur in einer Ansicht gemäß Fig. 2,
Fig. 4 eine weitere Ausgestaltung einer Soft-Touch-Polsterstruktur in einer Ansicht gemäß den Figuren 2 oder 3, und
Fig. 5 eine erfindungsgemäß schlauchförmige Soft-Touch-Polsterstruktur in einer Stirnseitenansicht.

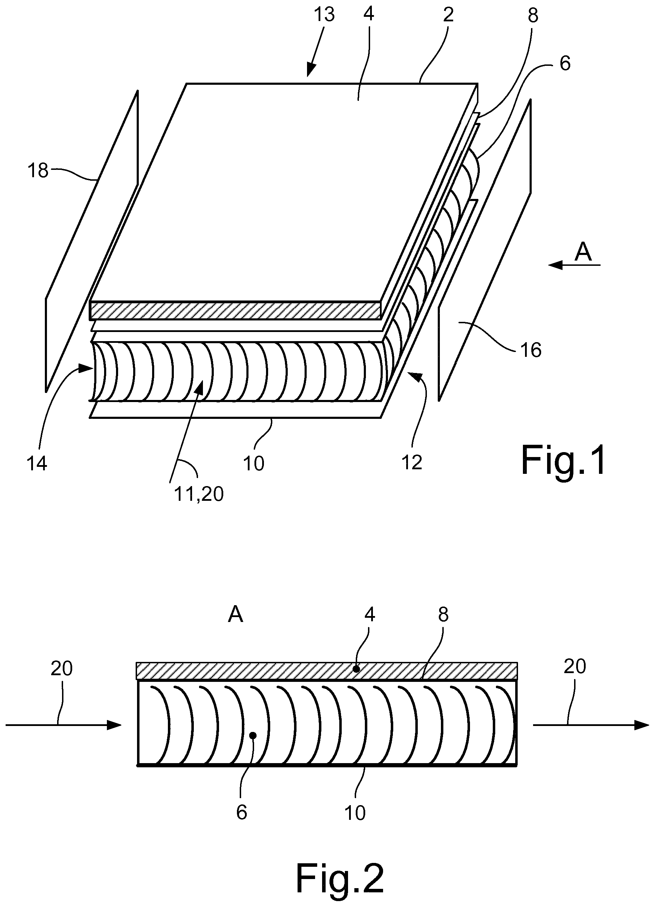

Die in Fig. 1 dargestellte Soft-Touch-Polsterstruktur ist als ein bahnförmiger Polsterstrukturabschnitt 2 ausgebildet, wie er in ähnlicher Form im Stand der Technik vorhanden ist. Soweit nicht schlauchförmig ausgebildet, gehört die Polsterstruktur gemäß Fig. 1 nicht zur Erfindung. Der Polsterabschnitt umfasst eine Obermaterialschicht 4, die vorzugsweise aus PUR, PVC, TPO, Leder, Textilware oder dergleichen besteht, und eine Untermaterialschicht 6, die vorzugsweise aus einem Abstandsgewirke, einem offenporigen Schaum oder einer Vliesstruktur besteht. Die Obermaterialschicht 4 und die Untermaterialschicht 6 werden beim Zusammenbau mittels einer zwischen diesen beiden Materialschichten 4, 6 angeordneten Klebeschicht 8 miteinander verbunden. Wie bereits weiter oben ausgeführt wurde, kann die Obermaterialschicht 4 mit der Untermaterialschicht 6 auch mittels eines Kaschierverfahrens verbunden werden. An der Unterseite der Untermaterialschicht 6 ist eine gasundurchlässige Wandfolie 10 angeordnet, welche die Untermaterialschicht 6 nach unten gasdicht verschließt.

Der in Fig. 1 rechte seitliche Stirnseitenbereich 12 und der linke seitliche Stirnseitenbereich 14 der Untermaterialschicht 6 können wie dargestellt ebenfalls jeweils durch eine Wandfolie 16, 18 gasundurchlässig verschlossen werden. Die nicht verschlossenen Stirnseitenbereiche 11 und 13 dienen als Zugangs- beziehungsweise Ausgangsöffnungen für ein gasförmiges Medium. Auf diese Weise bildet die Untermaterialschicht 6 eine Art Kanal, welcher in der Schichtebene des Untermaterials 6 beispielsweise in Richtung der Strömungsrichtung 20 von dem gasförmigen Medium durchströmt werden kann.

Die untere Wandfolie 10 und die beiden seitlichen Wandfolien 16, 18 können mit der Untermaterialschicht 6 durch Kleben verbunden werden. Diese drei Wandfolien 10, 16, 18 können auch gemeinsam einstückig ausgebildet sein.

Fig. 2 zeigt den Polsterstrukturabschnitt 2 der Fig. 1 in einer Seitenansicht A gemäß Fig. 1, wobei die rechte seitliche Wandfolie 16 fortgelassen wurde. Die Untermaterialschicht 6 ist mit der Obermaterialschicht 4 über die Klebeschicht 8 weitgehend gasundurchlässig verbunden und durch die Wandfolie 10 nach unten verschlossen. Die Untermaterialschicht 6 bildet auf diese Weise einen Kanal, durch den ein gasförmiges Kühlmedium bzw. Heizmedium in der Strömungsrichtung 20 hindurchleitbar ist, so dass die Obermaterialschicht 4 in gewünschter Weise temperiert werden kann.

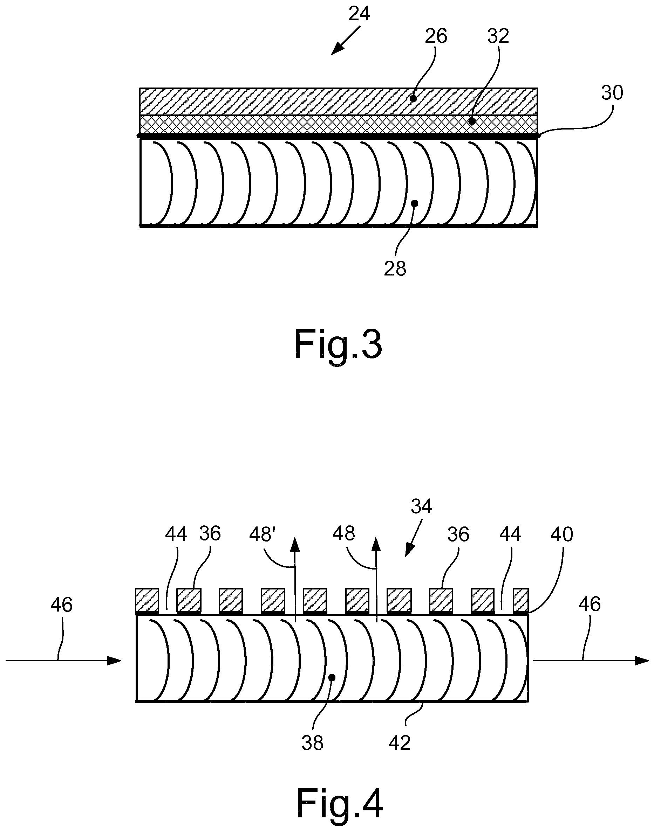

Fig. 3 zeigt ein Ausführungsbeispiel eines Polsterstrukturabschnittes 24 etwa gemäß Fig. 2, wobei jedoch zwischen der Obermaterialschicht 26 und der Untermaterialschicht 28 mittels einer Klebeschicht 30 eine Textilträgerschicht 32 angeordnet ist, die ihrerseits mit der Obermaterialschicht 26 verbunden ist. Die Textilträgerschicht 32 besteht beispielsweise aus Baumwoll-Polyester-Material, einem Polyester-Material, einem Vlies oder einer Polyolefin-Folie.

Fig. 4 zeigt ein weiteres Ausführungsbeispiel eines Polsterstrukturabschnittes 34 etwa gemäß Fig. 2, bestehend aus einer Obermaterialschicht 36, die mit der Untermaterialschicht 38 mittels einer Klebeschicht 40 verbunden sowie durch eine Wandfolie 42 nach unten verschlossen ist. Die Obermaterialschicht 36 sowie die Klebeschicht 40 sind perforiert, wie mittels der Perforationsöffnungen 44 angedeutet ist, so dass zumindest ein Teil des in Strömungsrichtung 46 strömenden gasförmigen Mediums als diffuse Belüftungsströmung 48, 48' durch die Obermaterialschicht 36 austreten kann.

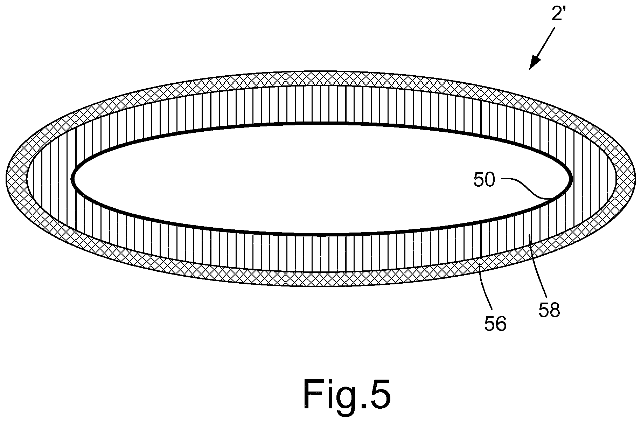

Fig. 5 zeigt eine erfindungsgemäß aufgebaute Soft-Touch-Polsterstruktur in der Ausführungsform einer schlauchförmigen Polsterstruktur 2', die aufgrund ihrer Geometrie ohne gasundurchlässige Seitenwände auskommt. Bei dieser Polsterstruktur 2' ist daher von radial außen nach radial innen gesehen zunächst die gasundurchlässige Obermaterialschicht 56, dann die gasdurchlässige Untermaterialschicht 58, und schließlich die gasundurchlässige Wandfolie 50 angeordnet. Eine solche schlauchförmigen Polsterstruktur 2' kann beispielsweise vorteilhaft zum Bespannen einer Rückenlehne eines Fahrzeugsitzes verwendet werden.

Die erfindungsgemäße Polsterstruktur ermöglicht in konstruktiv und baulich einfacher Weise einen weitgehend verlustfreien Wärme- oder Kälteübergang vom gasförmigen Medium auf die Obermaterialschicht. Insbesondere im Automobilbau lässt sie sich einfach in bestehende Sitzkonstruktionen integrieren, womit sich alle Anforderungen hinsichtlich Kühlung, Heizung und Belüftung des Fahrzeuges erfüllen lassen. Dabei wird der Sitzkomfort in keiner Weise eingeschränkt, da die mechanischen Eigenschaften der Polsterstruktur nicht beeinträchtigt werden.

### Bezugszeichen

- 2: Bahnförmige Polsterstruktur; Polsterstrukturabschnitt
- 2': Schlauchförmige Polsterstruktur; Polsterstrukturabschnitt
- 4: Obermaterialschicht
- 6: Untermaterialschicht
- 8: Klebeschicht
- 10: Untere Wandfolie
- 11: Einströmseitiger Stirnseitenbereich
- 12: Verschlossener rechter Stirnseitenbereich
- 13: Ausströmseitiger Stirnseitenbereich
- 14: Verschlossener linker Stirnseitenbereich
- 16: Erste seitliche Wandfolie
- 18: Zweite seitliche Wandfolie
- 20: Strömungsrichtung
- 24: Polsterstrukturabschnitt
- 26: Obermaterialschicht
- 28: Untermaterialschicht
- 30: Klebeschicht
- 32: Textilträgerschicht
- 34: Polsterstrukturabschnitt
- 36: Obermaterialschicht
- 38: Untermaterialschicht
- 40: Klebeschicht
- 42: Untere Wandfolie
- 44: Perforationsöffnungen
- 46: Strömungsrichtung
- 48: Belüftungsströmung
- 48': Belüftungsströmung
- 50: Untere Wandfolie
- 56: Obermaterialschicht
- 58: Untermaterialschicht
- A: Seitenansicht

## Patentansprüche

1. Mehrschichtige Soft-Touch-Polsterstruktur (2, 2'), welche eine im Wesentlichen gasundurchlässige Obermaterialschicht (4, 26, 36, 56) und zumindest eine unter dieser angeordnete voluminöse, druckelastische Untermaterialschicht (6, 28, 38, 58) aufweist, die Untermaterialschicht (6, 28, 38, 58) aus einem gasdurchlässigen Material besteht, wobei die Unterseite der Untermaterialschicht (6, 28, 38, 58) mit einer gasundurchlässige Wandfolie (10, 42, 50) verschlossen ist, und wobei in den einströmseitigen Stirnseitenbereich (11) sowie in dem ausströmseitigen Stirnseitenbereich (13) der Untermaterialschicht (6, 28, 38, 58) Zugangs- und Ausgangsöffnungen für ein durch die Untermaterialschicht (6, 28, 38, 58) hindurch zu leitendes gasförmiges Kühlmedium beziehungsweise Heizmedium vorgesehen sind **dadurch gekennzeichnet, dass** die Polsterstruktur eine schlauchförmige Geometrie aufweist, wobei von radial außen nach radial innen gesehen zunächst die Obermaterialschicht (56), dann die Untermaterialschicht (58) und schließlich die gasundurchlässige Wandfolie (50) angeordnet ist.

2. Polsterstruktur (2) nach Anspruch 1, **dadurch gekennzeichnet, dass** die nicht als Zugangsöffnungen beziehungsweise Ausgangsöffnungen vorgesehenen seitlichen Stirnseitenbereiche (12, 14) der Untermaterialschicht (6) gasdicht verschlossen sind.

3. Polsterstruktur (2) nach Anspruch 2, **dadurch gekennzeichnet, dass** die seitlichen Stirnseitenbereiche (12, 14) jeweils mittels einer gasundurchlässigen Wandfolie (16, 18) verschlossen sind.

4. Polsterstruktur (2, 2') nach einem der Ansprüche 1 bis 3, **dadurch gekennzeichnet, dass** die Obermaterialschicht (4, 26, 36, 56) aus Polyurethane (PUR), Polyvinylchlorid (PVC), Polyolefin (TPO), Leder oder Textilware besteht.

5. Polsterstruktur (2, 2') nach einem der Ansprüche 1 bis 4, **dadurch gekennzeichnet, dass** die Untermaterialschicht (6, 28, 38, 58) aus einem Abstandsgewirke, einem offenporigen Schaumstoff oder einer Vliesstruktur besteht.

6. Polsterstruktur (2, 2') nach einem der Ansprüche 1 bis 5, **dadurch gekennzeichnet, dass** die Obermaterialschicht (4, 26, 36, 56) und/oder eine gasundurchlässige Wandfolie (10) mit der Untermaterialschicht (6, 28, 38, 58) jeweils durch Kaschieren oder Kleben verbunden sind.

7. Polsterstruktur (2, 2') nach einem der Ansprüche 1 bis 6, **dadurch gekennzeichnet, dass** zwischen der Obermaterialschicht (4, 26, 36, 56) und der Untermaterialschicht (6, 28, 38, 58) eine Textilträgerschicht (32) aus Polyester (PES), aus Baumwolle/Polyester, aus Vlies oder aus Polyolefin-Folie (TPO-Folie) angeordnet ist.

8. Polsterstruktur (2, 2') nach einem der Ansprüche 1 bis 7, **dadurch gekennzeichnet, dass** die an der Oberseite der Untermaterialschicht (6, 28, 38, 58) angeordnete Schicht, bestehend aus Obermaterialschicht (4, 26, 56) und Textilträgerschicht (32), perforiert ist.

## Claims

1. Multi-layered soft-touch upholstery structure (2, 2') which has a substantially gas-impermeable upper material layer (4, 26, 36, 56) and, disposed therebelow, at least one voluminous pressure-elastic lower material layer (6, 28, 38, 58), the lower material layer (6, 28, 38, 58) being composed of a gas-permeable material, wherein the lower side of the lower material layer (6, 28, 38, 58) is closed off by way of a gas-impermeable wall film (10, 42, 50), and wherein entry and exit openings for a gaseous cooling medium or heating medium, respectively, that is to be directed through the lower material layer (6, 28, 38, 58) are provided in the inflow-side end side region (11) as well as in the outflow-side end side region (13) of the lower material layer (6, 28, 38, 58),
**characterized in that**
the upholstery structure has a tubular geometry wherein, when viewed from radially outside to radially inside, the upper material layer (56) is disposed first, then the lower material layer (58), and finally the gas-impermeable wall film (50).

2. Upholstery structure (2) according to Claim 1,
**characterized in that**
the lateral end side regions (12, 14) of the lower material layer (6) that are not provided as entry openings or exit openings, respectively, are closed in a gas-tight manner.

3. Upholstery structure (2) according to Claim 2,
**characterized in that**
the lateral end side regions (12, 14) are in each case closed by means of a gas-impermeable wall film (16, 18).

4. Upholstery structure (2, 2') according to one of Claims 1 to 3,
**characterized in that**
the upper material layer (4, 26, 36, 56) is composed of polyurethane (PUR), polyvinylchloride (PVC), polyolefin (TPO), leather, or textile goods.

5. Upholstery structure (2, 2') according to one of Claims 1 to 4,
**characterized in that**
the lower material layer (6, 28, 38, 58) is composed of a woven warp-knitted or weft-knitted spacer fabric, an open-pore foam material, or a non-woven structure.

6. Upholstery structure (2, 2') according to one of Claims 1 to 5,
**characterized in that**
the upper material layer (4, 26, 36, 56) and/or a gas-impermeable wall film (10) are/is in each case connected to the lower material layer (6, 28, 38, 58) by laminating or adhesive bonding.

7. Upholstery structure (2, 2') according to one of Claims 1 to 6,
**characterized in that**
a textile carrier layer (32) from polyester (PES), from cotton/polyester, from non-woven, or from polyolefin film (TPO film) is disposed between the upper material layer (4, 26, 36, 56) and the lower material layer (6, 28, 38, 58).

8. Upholstery structure (2, 2') according to one of Claims 1 to 7,
**characterized in that**
the layer which is composed of the upper material layer (4, 26, 56) and the textile carrier layer (32) and which is disposed on the upper side of the lower material layer (6, 28, 38, 58) is perforated.

## Revendications

1. Structure de rembourrage multicouche (2, 2') douce au toucher qui comprend une couche de matière supérieure (4, 26, 36, 56) sensiblement imperméable aux gaz et au moins une couche de matière inférieure (6, 28, 38, 58) volumineuse et élastique par compression, laquelle couche de matière inférieure (6, 28, 38, 58) est formée d'une matière perméable aux gaz,
le côté inférieur de la couche de matière inférieure (6, 28, 38, 58) étant fermée par un film de paroi (10, 42,50) imperméable aux gaz, et
des ouvertures d'accès et de sortie destinées à un milieu chauffant ou un milieu de refroidissement gazeux devant passer à travers la couche de matière inférieure (6, 28, 38, 58) étant ménagées dans la région d'extrémité côté entrée (11) et dans la région d'extrémité côté sortie (13) de la couche de matière inférieure (6, 28, 38, 58), **caractérisée en ce que** la structure de rembourrage présente une géométrie tubulaire, la couche de matière supérieure (56), la couche de matière inférieure (58) et le film de paroi (50) imperméable aux gaz étant disposés dans cet ordre lorsque l'on regarde dans une direction allant d'une position radialement extérieure à une position radialement intérieure.

2. Structure de rembourrage (2) selon la revendication 1, **caractérisée en ce que** les régions d'extrémité latérale (12, 14) de la couche de matière inférieure (6) qui ne sont pas pourvues d'ouvertures d'accès ou d'ouvertures de sortie sont fermées de manière étanche aux gaz.

3. Structure de rembourrage (2) selon la revendication 2, **caractérisée en ce que** les régions d'extrémité latérales (12, 14) sont fermées chacune par un film de paroi (16, 18) imperméable aux gaz.

4. Structure de rembourrage (2, 2') selon l'une des revendications 1 à 3, **caractérisée en ce que** la couche de matière supérieure (4, 26, 36, 56) est en polyuréthanes (PUR), en polychlorure de vinyle (PVC), en polyoléfine (TPO), en cuir ou en produit textile.

5. Structure de rembourrage (2, 2') selon l'une des revendications 1 à 4, **caractérisée en ce que** la couche de matière inférieure (6, 28, 38, 58) est formée d'un tissu maillé d'espacement, d'une matière alvéolaire à pores ouverts ou d'une structure non tissée.

6. Structure de rembourrage (2, 2') selon l'une des revendications 1 à 5, **caractérisée en ce que** la couche de matière supérieure (4, 26, 36, 56) et/ou un film de paroi (10) imperméable aux gaz sont reliés chacun à la couche de matière inférieure (6, 28, 38, 58) par collage ou contre-collage.

7. Structure de rembourrage (2, 2') selon l'une des revendications 1 à 6, **caractérisée en ce qu'**une couche de support textile (32) en polyester (PES), en coton/polyester, en non-tissé ou en film polyoléfine (film TPO) est disposée entre la couche de matière supérieure (4, 26, 36, 56) et la couche de matière inférieure (6, 28, 38, 58).

8. Structure de rembourrage (2, 2') selon l'une des revendications 1 à 7, **caractérisée en ce que** la, couche, disposée sur un côté supérieur de la couche de matière inférieure (6, 28, 38, 58) et formée de la couche de matière supérieure (4, 26, 56) et de la couche de support textile (32), est perforée.
